# EUROPEAN PATENT SPECIFICATION

(11) **EP 0 958 513 B1**
(45) Date of publication and mention of the grant of the patent: **20.08.2003**
(21) Application number: 98957629.3
(22) Date of filing: 05.11.1998
(51) Int. Cl.: A61F 2/16, G02B 3/10

(54) **METHOD OF REDUCING GLARE ASSOCIATED WITH MULTIFOCAL OPHTHALMIC LENSES**
VERFAHREN ZUR VERRINGERUNG DER BLENDUNG BEI MULTIFOKALEN OPHTHALMISCHEN LINSEN
PROCEDE PERMETTANT DE REDUIRE L'EBLOUISSEMENT ASSOCIE AUX LENTILLES PROGRESSIVES

(30) Priority: 04.12.1997 US 984909
(43) Date of publication of application: 24.11.1999
(73) Proprietor: ALCON LABORATORIES, INC., Fort Worth Texas 76134-2099 (US)
(72) Inventor: PATEL, Anilbhai, S., Arlington, TX 76017 (US); GRAHAM, William, M., Fort Worth, TX 76116 (US)
(74) Representative: Hanna, Peter William Derek
(86) International application number: PCT/US98/23646
(87) International publication number: WO 99/028769

(56) References cited:
- EP-A- 0 756 183
- EP-A- 0 855 602
- US-A- 5 120 120
- US-A- 5 470 932
- US-A- 5 617 154
- DATABASE WPI Section Ch, Week 9313 Derwent Publications Ltd., London, GB; Class A14, AN 93-105007 XP002094520 & JP 05 045610 A (TOKYO KEIKAKU KK) , 26 February 1993
- DATABASE WPI Section Ch, Week 9047 Derwent Publications Ltd., London, GB; Class A14, AN 90-352116 XP002094521 & JP 02 254402 A (HOYA CORP) , 15 October 1990
- PATENT ABSTRACTS OF JAPAN vol. 095, no. 002, 31 March 1995 & JP 06 324293 A (DAISERU AMIBOSHI SANGYO KK), 25 November 1994

## Description

### Background of the Invention

This invention relates generally to the field of intraocular lenses and, more particularly, to bifocal, varifocal or multifocal intraocular lenses (IOLs).

The human eye in its simplest terms functions to provide vision by transmitting light through a clear outer portion called the cornea, and focusing the image by way of a lens onto a retina. The quality of the focused image depends on many factors including the size and shape of the eye, and the transparency of the cornea and lens.
When age or disease causes the lens to become less transparent, vision deteriorates due to an inadequate image or by the scattered and diminished light which can be transmitted to the retina. This deficiency in the lens of the eye is medically known as a cataract. An accepted treatment for this condition is surgical removal of the lens and replacement of the lens function by an IOL.

The majority of ophthalmic lenses, including contact lenses and IOLs, currently used are of a monofocal design, (*i.e*., having one fixed focal length). The focal length of the implanted IOL generally is chosen to optimize distance vision at 3 meters from the patient. Most patients receiving an IOL still require glasses for clear distance and near vision.

Various multifocal ophthalmic lens designs are currently under investigation and these designs generally fall into one of two categories, refractive lenses and diffractive lenses. Refractive lenses are more fully described in U.S. Patent Nos.5,147,393, 5,217,489 (Van Noy, et al.), U.S. Patent No. 5,152,787 (Hamblen), U.S. Patent No. 4,813,955 (Achatz, et al.), U.S. Patent Nos. 5,089,024, 5,112,351 (Christie, et al.), U.S. Patent Nos. 4,769,033. 4,917,68, 5,019,099, 5,074,877, 5,236,452, 5,326,348 (Nordan), 5,192,318, 5,366,500 (Schneider, et al.), U.S. Patent Nos. 5,139,519, 5,192,317 (Kalb), U.S. Patent No. 5,158,572 (Neilsen), U.S. Patent No. 5,507,806 and PCT Publication No. WO 95/31156 (Blake) and U.S. Patent No. 4,636,211 (Nielsen, et al.). Diffractive lenses use nearly periodic microscopic structures on the lens to diffract light into several directions simultaneously. This is similar to a diffraction grating and the multiple diffraction orders focus the light into various images corresponding to different focal lengths of the lens. Diffractive multifocal contact lenses and lOLs are more fully discussed in U.S. Patent No. 5,178,636 (Silberman), U.S. Patent Nos. 4,162,122, 4,210,391, 4,338,005, 4,340,283, 4,995,714, 4,995,715, 4,881,804, 4,881,805, 5,017,000, 5, 054, 905, 5,056,908, 5,120,120, 5,121,979, 5,121,980, 5,144,483, 5,117,306 (Cohen), U.S. Patent Nos. 5,076,684, 5,116,111 (Simpson, et al.), U.S. Patent No. 5,129,718 (Futhey, et al.) and U.S. Patent Nos. 4,637,697, 4,641,934 and 4,655,565 (Freeman).

While a diffractive IOL may have a number of focal lengths, generally, IOLs with only two focal lengths (far and near) are the most common. As with any simultaneous vision multi focal lens, a defocused image (or images) is superimposed (on the retina) on the focused component because of the second lens power, but the defocused image is rarely observed by the user, who concentrates on the image of interest. The defocused image acts as a veiling glare source and thus interferes and degrades the focused image. Under certain circumstances (for example, at night), the pupil diameter of the user can expand to 5 millimeters (mm) or more, and a discrete distant light source (*e.g*., automobile headlights or street lights) can appear to be surrounded by a "halo" or "rings". A significant component of the halo is caused by the light that is directed to the near image which becomes defocused at the retina. The visibility of the halo is affected by the diameter of the lens region directing light to the near image, the proportion of total energy directed to the near image, and the overall imaging aberrations of the eye.

In U.S. Patent No. 4,881,805, Cohen suggests that the intensity of light traveling through a diffractive lens can be varied by reducing the echelette depth at the lens periphery, thus reducing glare (column 4, lines 63-68). Cohen further states that the zone boundary radii of the diffractive zones need to obey the formula:${\text{R}}_{\text{m}} \text{=} \sqrt{\text{2} \text{mwf}}$ where:
w = the wavelength of light
m = integer representing the m^{th} zone
f = focal length of the 1^{st} order diffraction

### Column 5, lines 17-31.

Cohen's theory states that the glare results from the depth of the steps at the diffractive zone boundaries may be more applicable to contact lenses than intraocular lenses. Contact lenses generally move on the eye and the grooves can become filled with debris. In addition, the additive power of the contact lenses generally is less than that of intraocular lenses, which puts the defocused image more in focus, and also the patient's natural residual accommodation may alter the visibility of glare or halos.

U.S. Patent Nos. 5,470,932 and 5,662,707 (Jinkerson), disclose the use of yellow dyes in ophthalmic lenses to block or lower the intensity of near UV and blue light (between 300 nanometers and 500 nanometers) that passes through the lens. Near UV and blue light is believed to be hazardous to the retina, and including blue-blocking dyes in the IOL is believed to restore the retinal protection lost when the natural lens is removed.

Suitable blue light-blocking dyes disclosed include acrylates/methacrylates of the formula: 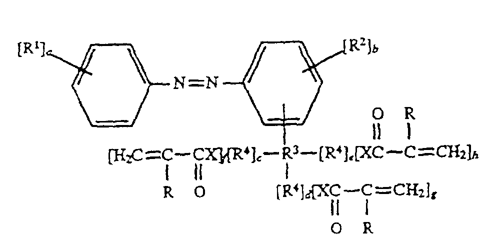 wherein
R=H or CH₃;
R¹=H, C₁ to C₂₀ alkyl, OCH₃, OC₂H₅, OC₃H₇, or OC₄H₉;
a and b independently=the integer 1 or 2;
R²=R¹, OH, NH₂, NHR⁵, N(R⁵)₂, SH, SR⁵, OR⁵, OSi(R⁵)₃, or Si(R⁵)₃;
R³ is directly attached to the dye moiety and consists of an alkyl group of up to 6 carbon atoms;
R⁴ is an acylic organic spacing group of up to 10 atoms which is composed of carbon, hydrogen, silicon, oxygen, nitrogen, phosphorous, sulfur, chlorine, bromine, or fluorine alone or in any combination;
X=O, NH, NR⁵;
R⁵=C₁ to C₁₀ alkyl;
d, e, g, and h independently=an integer from 0 to 4; and c and f independently=an integer from 1 to 4.

Electron-withdrawing groups are not permitted to be covalently bonded to the dye moiety because they can weaken the strength of the yellow dye and, in some cases, change the absorption nature of the dye sufficiently to cause a change in color. Examples of electron-withdrawing groups which are not permitted to be directly attached to the dye moiety include carbonyl groups, such as those found in ketones; carboxylic acid esters; amides; imines; immides; imminic acid esters (especially analogues derived from 1,3,5-triazeno systems); ureas; urethanes; and so on.

An example given is N-2-[3-(2'-methylphenylazo)-4-hydroxyphenyl]ethyl methacrylamide.

Further suitable blue light-blocking dyes disclosed by Jinkerson include diacrylates/dimethacylates of the formula: 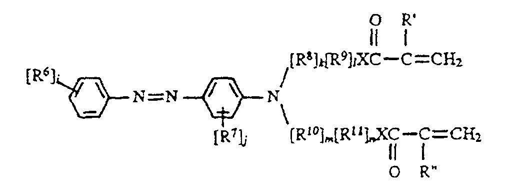 wherein
R¹ and R" independently=H or CH₃;
R⁶ and R⁷ independently=R¹;
i and j independently=the integer 1 or 2;
R⁸, R⁹, R¹⁰ and R¹¹ independently=R⁴;
k and m independently=an integer from 1 to 6;
l and n independently=an integer from 0 to 6;
x=O, NH, NR⁵; and
R⁵=C₁ to C₁₀ alkyl.

An example given is N, N-bis-(2-methacroylethyl)-(4-phenyl-azo)aniline.

US-A-5,120,120 (Cohen) describes a diffractive multifocal phase zone contact lens or intraocular lens including at least two annular zones disposed concentrically about the optical axis and spaced apart in a radial dimension from the optical axis. An absorbing means is disposed on a portion of selected annular zones to absorb only a portion of the incident light. The purpose of the absorbing means is preferentially to reduce the light that would otherwise be focused to the higher order focal points. The absorbing means is disposed on the annular zones to absorb incident light non-uniformly along the radial dimension, and preferably according to a repetitive pattern. The echellettes of the diffractive lens provide a plurality of focal points, focal orders or orders of diffraction. Diffractive orders higher than order 1 are considered subsidiary, spurious or higher diffractive orders. Only orders 0 and 1 are used as the primary diffractive orders because these two orders receive 40.5% of the transmitted light. The purpose of the absorbing means is to filter out 100% of the higher diffractive orders. To accomplish this, the absorbing means is disposed only on the annular zones of the lens rather than across the whole lens to create a pattern of varying transmissivity across the lens, creating a transmission profile for the lens.

US-A-5,617,154 (Hoffman) describes a light-absorbing monofocal contact lens, or intraocular lens, tinted with colorants to filter out ultraviolet and blue light wavelengths of from about 200 to about 500 nm. This provides the desired spectral transmittance characteristics over the tinted portion of the lens, and may be chosen by selecting colorant combinations to fill the particular needs of a wearer, for example for limiting the transmittance of visible light in high intensity light environments. Such a lens affords the wearer protection against macular degeneration and other diseases of the eye caused by overexposure to ultraviolet radiation.

Prior to the present invention, there has been no recognition in the multifocal lens art of selectively using near UV and blue light blocking dyes to reduce the glare and halos that can be associated with multifocal IOLs.

Accordingly, a need continues to exist for a multifocal IOL that minimizes glare or halos.

### Brief Summary of the Invention

The present invention provides a method of reducing or eliminating the glare or halo associated with multifocal optics in a multifocal intraocular lens, and a method of manufacture of same, in accordance with the claims which follow.

### Brief Description of the Drawings

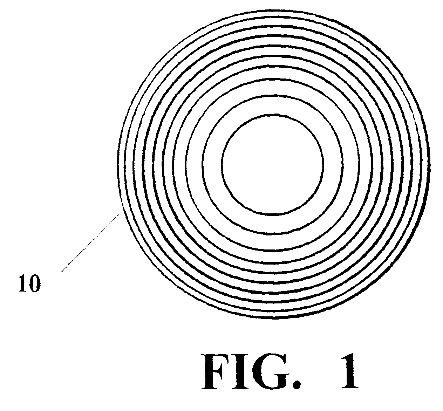

FIG. 1 is a plan view of a diffractive ophthalmic lens that may be used with the present invention.

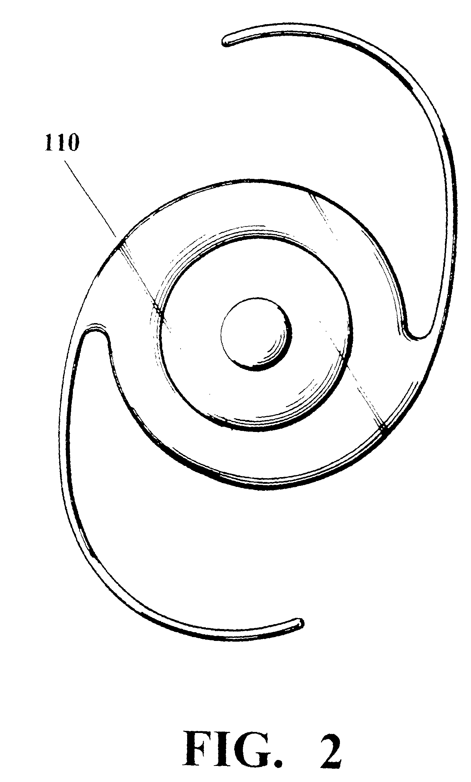

FIG. 2 is a plan view of a refractive ophthalmic lens that may be used with the present invention.

### Detailed Description of the Invention

The inventors have discovered that including a near UV and/or blue light blocking dye or dyes in multifocal lens 10 or 110 reduces or eliminates the glare and halos associated with multifocal optics. One likely mechanism of action is that the Rayleigh scattering of light sources within the line of sight of the focused and defocused image by the cornea and the lens is disproportionate among the transmission band, with short wavelengths being scattered more than longer wavelengths (Rayleigh's Law holds that the scattering intensity is proportional to λ⁻⁴ where λ is the wavelength). Thus, the filtering of the shorter wavelengths reduces this scattering as well as reducing chromatic aberrations. One other mechanism is based on the recognition in the lighting industry that the discomfort associated with a glare source not in the line of sight may be predicted using the following equation:${\text{glare sensation = (B}}_{\text{s}} {}^{\text{n}} {\text{/B}}_{\text{b}} {}^{\text{c}} {\text{) * (ω}}^{\text{b}} {\text{/ρ}}^{\text{d}} \text{)}$ where
Bₛ = luminace of the glare source
B_{b} = luminance of the background
ω = angular subtense of the glare source
ρ = deviation of glare source from line of sight
Constants n, b, c and d for predicting glare sensation caused by glare sources away from line of sight (*e.g*. headlights of automobiles in neighboring lanes or street lights) is not solidly established. Durrant (1977) established the values as n = 1.6, b = 0.8, c = 1 and d = 1.6. Using these values, a twenty-five percent reduction in the luminance for a lower wavelength of the glare source and background source luminance outside of the line of sight results in nearly a fifty-six percent reduction in glare sensation contributed by the lower wavelength.

All multifocal lOLs based on simultaneous vision optics have veiling, Rayleigh scattering law abiding, glare from out of focus images of light sources within the line of sight as well as glare effects of light sources not in the line of sight, especially during night driving when the pupil is large. The blue blocking and/or near UV blocking multifocal IOL of the present invention thus addresses both types of glare and improves upon the prior art by selectively filtering out the lower wavelengths from approximately 400 nanometers up to approximately 550 nanometers.

Any multifocal lens 10 or 110 can be used in the present invention and lens 10 or 110 may be made from any suitable material such as polymethylmethacrylate, silicone, soft acrylic or HEMA. Preferably, the blue-blocking dye used will be covalently bonded in the material used to make lens 10 or 110 and will be non-leaching. For example, if lens 10 or 110 is a soft acrylic lens, the dyes disclosed in U.S. Patent Nos. 5,470,932 and 5,662,707 (Jinkerson) and described above may be used.

This description is given for purposes of illustration and explanation. It will be apparent to those skilled in the relevant art that modifications may be made to the invention as herein described without departing from its scope. For example, the exact wavelengths that must be filtered out as well as the extent of the attenuation will vary by the lens material used. Therefore dyes that block out wavelengths below 410 nanometers, below 420 nanometers, below 430 nanometers, below 440 nanometers, below 450 nanometers, below 460 nanometers, below 470 nanometers, below 480 nanometers, below 490 nanometers, below 500 nanometers, below 510 nanometers, below 520 nanometers, below 530 nanometers, below 540 nanometers and below 550 nanometers may all be suitable for use with the present invention.

## Claims

1. A method of reducing the glare or halos associated with multifocal optics in a multifocal intraocular lens (10,110), comprising:
incorporating a non-leaching dye into a material used to make the lens, the dye suitable for selectively filtering out wavelengths of light between approximately 550 nanometers and approximately 400 nanometers from sources within a line of sight of the eye, when said intraocular lens is implanted in the eye.

2. The method of claim 1, incorporating only a blue light blocking dye in the lens (10,110).

3. The method of claim 1, incorporating only a near UV blocking dye in the lens (10,110).

4. The method of any one of claims 1 to 3, wherein the multifocal intraocular lens is a diffractive lens (10).

5. The method of any one of claims 1 to 3, wherein the multifocal intraocular lens is a refractive lens (110).

6. A method of manufacturing a multifocal intraocular lens (10,110), comprising:
incorporating a non-leaching dye into a material used to make the lens, the dye suitable for selectively filtering out wavelengths of light between approximately 550 nanometers and approximately 400 nanometers from sources within a line of sight of the eye, when said intraocular lens is implanted in the eye, for reducing the glare or halos associated with multifocal optics in said lens.

## Patentansprüche

1. Verfahren zur Verringerung der Blendung oder der Lichthöfe, die mit multifokalen Optiken in einer multifokalen intraokularen Linse (10, 110) verknüpft sind, das folgendes umfaßt:
Einbringen eines nicht auslaugenden Farbstoffs in ein Material, das zum Herstellen der Linse benutzt wird, wobei der Farbstoff zum selektiven Herausfiltern von Lichtwellenlängen zwischen etwa 550 nm und etwa 400 nm aus Quellen in einer Sichtlinie des Auges geeignet ist, wenn die intraokulare Linse im Auge implantiert ist.

2. Verfahren nach Anspruch 1, bei dem nur ein blaues Licht blockierender Farbstoff in die Linse (10, 110) eingebracht wird.

3. Verfahren nach Anspruch 1, bei dem nur ein nahes UV blockierender Farbstoff in die Linse (10, 110) eingebracht wird.

4. Verfahren nach einem der Ansprüche 1 bis 3, bei dem die multifokale intraokulare Linse eine lichtbeugende Linse (10) ist.

5. Verfahren nach einem der Ansprüche 1 bis 3, bei dem die multifokale intraokulare Linse eine lichtbrechende Linse (110) ist.

6. Verfahren zum Herstellen einer multifokalen intraokularen Linse (10, 110), das folgendes umfaßt:
Einbringen eines nicht auslaugenden Farbstoffs in ein Material, das zum Herstellen der Linse benutzt wird, wobei der Farbstoff zum selektiven Herausfiltern von Lichtwellenlängen zwischen etwa 550 nm und etwa 400 nm aus Quellen in einer Sichtlinie des Auges geeignet ist, wenn die intraokulare Linse im Auge implantiert ist, zum Verringern der Blendung oder der Lichthöfe, die mit einer multifokalen Optik in der Linse verknüpft sind.

## Revendications

1. Procédé pour réduire le reflet ou des halos associés à des éléments optiques multifocaux dans une lentille intraoculaire multifocale (10, 110), comportant :
l'incorporation d'un colorant non-lixiviant dans un matériel utilisé pour fabriquer la lentille, le colorant étant adapté pour filtrer de manière sélective des longueurs d'onde de lumière entre approximativement 550 nanomètres et approximativement 400 nanomètres provenant de sources dans une ligne de visée de l'oeil, lorsque ladite lentille intraoculaire est implantée dans l'oeil.

2. Procédé selon la revendication 1, comportant uniquement un colorant bloquant une lumière bleue dans la lentille (10, 110).

3. Procédé selon la revendication 1, comportant uniquement un colorant bloquant des rayons proches UV dans la lentille (10, 110).

4. Procédé selon l'une quelconque des revendications 1 à 3, dans lequel la lentille intraoculaire multifocale est une lentille de diffraction (10).

5. Procédé selon l'une quelconque des revendications 1 à 3, dans lequel la lentille intraoculaire multifocale est une lentille de réfraction (110).

6. Procédé pour fabriquer une lentille intraoculaire multifocale (10, 110), comportant:
l'incorporation d'un colorant non-lixiviant dans un matériel utilisé pour fabriquer la lentille, le colorant étant adapté pour filtrer de manière sélective des longueurs d'onde de lumière entre approximativement 550 nanomètres et approximativement 400 nanomètres provenant de sources dans une ligne de visée de l'oeil, lorsque ladite lentille intraoculaire est implantée dans l'oeil, pour réduire le reflet ou des halos associés à des éléments optiques multifocaux de ladite lentille.
